# EUROPEAN PATENT APPLICATION

(11) **EP 4 807 587 A1**
(43) Date of publication of application: **16.09.2026**
(21) Application number: 25163065.3
(22) Date of filing: 11.03.2025
(51) Int. Cl.: G06F 17/17, G16C 10/00

(54) **PREPARING A SIMULATION OF A PHYSICAL SYSTEM**

(71) Applicant: HQS Quantum Simulations GmbH, 76131 Karlsruhe (DE)
(72) Inventor: Reiner, Jan-Michael, Karlsruhe (DE); Fratus, Keith R., Offenburg (DE); Marthaler, Michael, Karlsruhe (DE)
(74) Representative: Durm Patentanwälte PartG mbB

(57) **Abstract**

The present invention relates to an apparatus (12) for preparing a simulation of a physical system, comprising: an input interface (16) for receiving a description input including a system of differential equations or a construction specification of said system of differential equations, wherein said system of differential equations describes an evolution of an expectation value for an observable of interest of the physical system over time, and an accuracy parameter corresponding to a desired accuracy of the simulation; a graph unit (18) for determining a graph based on the description input, wherein a start vertex of the graph represents an expectation value of the observable of interest, further vertices of the graph represent coupled expectation values and connections between the vertices represent couplings between the expectation values; a distance unit (20) for determining a length parameter for each connection, said length parameter being determined based on an inverse of an absolute value of a coupling strength of the respective coupling; a restriction unit (22) for removing vertices from the graph based on the length parameters of connections between the start vertex and the respective vertex and based on the accuracy parameter to obtain a restricted graph; and a truncation unit (24) for deriving a truncated system of differential equations based on the restricted graph and the description input. The present invention further relates to a simulation system, a method and a computer program.

## Description

The present invention relates to an apparatus for preparing a simulation of a physical system. The present invention further relates to a simulation system as well as to a method and a computer program.

Computational simulations of physical phenomena or physical systems are widely used in physics, engineering and material sciences to model complex interactions in various domains, including quantum mechanics, nuclear magnetic resonance (NMR) spectroscopy, fluid dynamics, and electromagnetic field interactions. For studying such physical systems, researchers and engineers often need to analyze complex interactions of molecules, particles and other entities. Simulations thereby allow reducing the need for costly or impractical real-world experiments.

Many physical phenomena can be described mathematically by systems of differential equations, which are subsequently solved using numerical methods such as finite difference methods, finite element methods or spectral methods. In the domain of quantum mechanics, where the Schrödinger equation governs system behavior, or in electromagnetism, where Maxwell's equations describe wave propagation, solving these equations usually requires significant computational resources. The computational burden stems from the need to discretize continuous equations, store and manipulate large matrices, and perform iterative numerical approximations. The complexity is further exacerbated when simulating the time evolution of an observable, particularly when high accuracy over a finite time interval is required.

Several approaches have been developed to structure and optimize such simulations, in particular aiming at a reduction of the computational complexity.

In Rakovszky et al, "Dissipation-assisted operator evolution method for capturing hydrodynamic transport", 2020, an approach to calculating transport properties of strongly interacting lattice systems in the high temperature regime is disclosed. The approach is based on evolving observables in the Heisenberg picture and applying an artificial dissipation that reduces the weight on non-local operators. The observable is presented as a matrix product operator and it is shown that that the dissipation leads to a decay of operator entanglement, which allows capturing the dynamics to long times. The disclosed scheme is tested by calculating spin and energy diffusion constants in a variety of physical models. By gradually weakening the dissipation, the obtained results are extrapolated to the case of zero dissipation, thus estimating the physical diffusion constant with high precision.

White, "Effective dissipation rate in a Liouvillian-graph picture of high-temperature quantum hydrodynamics", 2023, relates to high temperature, generic strongly interacting spin systems. Such systems are expected to display hydrodynamics: local transport of conserved quantities, governed by classical partial differential equations like the diffusion equation. It is argued that the emergence of this dissipative long-wavelength dynamics from the system's unitary microscopic dynamics is controlled by the structure of the Liouvillian graph of the system's Hamiltonian, i.e., the graph induced on Pauli strings by commutation with that Hamiltonian. The Liouvillian graph decomposes naturally into subgraphs of Pauli strings of constant diameter, and the coherent dynamics of these subgraphs determines the rate at which operator weight spreads to long operators. This argument provides a quantitative theory of the emergence of a dissipative effective dynamics from unitary microscopic dynamics; it also leads to an effective model with Hilbert space dimension linear in system size and exponential in the UV cutoff for diffusion.

Despite these advancements, the fundamental challenge of determining the computational complexity of a simulation before execution and structuring input data in a way that optimally utilizes available resources, remains. Existing approaches primarily focus on refining numerical solvers or approximating solutions, but often lack systematic mechanisms for assessing and optimizing the preparatory phase of a simulation. This is particularly problematic when dealing with the time evolution of a system governed by a linear, first-order system of differential equations with many degrees of freedom, as is common in quantum mechanical systems and other complex physical models.

In view of this, it is an object of the present invention to provide an approach to systematically prepare input data for a simulation of a physical system which can be described by a system of differential equations. In particular, it is an object of the present invention to enable an accurate assessment of the minimal required computational resources for a simulation of a time evolution of a quantity or an observable of interest up to a given finite time, where the time evolution is governed by a linear, first-order system of differential equations of a number of degrees of freedom.

In a first aspect, the present invention relates to an apparatus for preparing a simulation of a physical system, comprising:
- an input interface for receiving a description input including a system of differential equations or a construction specification of said system of differential equations, wherein said system of differential equations describes an evolution of an expectation value for an observable of interest of the physical system over time, and an accuracy parameter corresponding to a desired accuracy of the simulation;
- a graph unit for determining a graph based on the description input, wherein a start vertex of the graph represents an expectation value of the observable of interest, further vertices of the graph represent coupled expectation values and connections between the vertices represent couplings between the expectation values;
- a distance unit for determining a length parameter for each connection, said length parameter being determined based on an inverse of an absolute value of a coupling strength of the respective coupling;
- a restriction unit for removing vertices from the graph based on the length parameters of connections between the start vertex and the respective vertex and based on the accuracy parameter to obtain a restricted graph; and
- a truncation unit for deriving a truncated system of differential equations based on the restricted graph and the description input.

In another aspect, the present invention relates to a simulation system for simulating a physical system, comprising an apparatus as defined above and a simulation unit for solving the truncated system of differential equations. Preferably, the system is configured to predict a nuclear magnetic resonance spectrum of a substance.

In another aspect, the present invention relates to a method for preparing a simulation of a physical system, comprising steps of:
- receiving a description input including a system of differential equations or a construction specification of said system of differential equations, wherein said system of differential equations describes an evolution of an expectation value for an observable of interest of the physical system over time, and an accuracy parameter corresponding to a desired accuracy of the simulation;
- determining a graph based on the description input, wherein a start vertex of the graph represents an expectation value of the observable of interest, further vertices of the graph represent coupled expectation values and connections between the vertices represent couplings between the expectation values;
- determining a length parameter for each connection, said length parameter being determined based on an inverse of an absolute value of a coupling strength of the respective coupling;
- removing vertices from the graph based on the length parameters of connections between the start vertex and the respective vertex and based on the accuracy parameter to obtain a restricted graph; and
- deriving a truncated system of differential equations based on the restricted graph and the description input.

In yet a further aspect of the present invention, a computer program is provided which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed apparatus, method, simulation system, computer program and medium have similar and/or identical preferred embodiments as the claimed apparatus and system, in particular as defined in the dependent claims and as disclosed herein.

The present invention is based on the idea of determining a graph representation of a system of differential equations. A system of differential equations or a construction specification for said system of differential equations such as a function is obtained along with a desired or required accuracy of the simulation. Based on this input, a graph is constructed, wherein vertices of the graph represent expectation values of observables of the physical system. Thereby, the graph starts with a start vertex that corresponds to an expectation value of the observable of interest, i.e., the desired quantity or parameter of which the behavior is to be simulated. Connections between the vertices are attributed a length parameter, which is particularly related to an inverse of a coupling strength of the corresponding couplings in the differential equations. Then, vertices of the graph are removed if their distance to the start vertex exceeds a limit which is determined based on the desired or required accuracy of the simulation, in particular the time horizon. Based on this, a truncated system of differential equations can be derived, which can then form the basis for computing the desired simulation.

Usually, a complete set of differential equations is too large as an input. Thus, a construction specification corresponding to a recipe or a function can be obtained (for example a Liouville-equation for quantum mechanical systems including the respective Hamiltonian). This construction specification may allow determining the derivative with respect to time of an expectation value and the further expectation values to which this expectation value is coupled in the differential equation. The graph unit may, based on this, derive which further vertices and connections have to be added, starting from the start vertex.

The approach of the present invention particularly makes it possible to assess the required computational resources for carrying out a simulation with a desired accuracy. For instance, it can be decided whether or not a simulation requires quantum computer hardware. In comparison to previous approaches, the present invention allows focusing on the most relevant degrees of freedom controlling the time evolution of the observable of interest. By making approximations in the space of operators rather than in physical space, the degree of approximation can be tuned very finely, while avoiding unnecessary degrees of freedom. Thereby, the accuracy of the approximate solution can be smoothly increased. In comparison to approximation methods which are based in physical space rather than operator space, the number of unnecessary degrees of freedom can be reduced. It is avoided that operators involving retained sites, regardless of whether these operators actually contribute to the dynamics in a meaningful way are accounted for. The computational complexity of a simulation can be reduced.

In a preferred embodiment, the input interface is configured to receive a description input including a Hamiltonian describing the physical system based on a plurality of interacting observables and an observable of interest of the plurality of interacting observables. In other words, the construction specification can correspond to a Hamiltonian (e.g., included in or together with the Liouville equation). This allows for an efficient processing and an efficient determination of the graph. An efficient data input can be obtained.

In a preferred embodiment, the input interface is configured to receive a description input including a quantum-mechanical Hamiltonian designed to model nuclear spin degrees of freedom in a molecule whose spectrum is to be studied in a nuclear magnetic resonance spectrum analysis. The approach of the present invention is particularly advantageous in the field of NMR spectrum analysis. In this application, usually a finite time evolution is relevant corresponding to a limited time of the experiment. The determination of irrelevant vertices in the graph can allow accounting for this and thereby, e.g., make it possible to simulate larger molecules on finite resources. In particular, a quantum-mechanical Hamiltonian designed to model the nuclear spin degrees of freedom in a molecule, whose spectrum is to be studied in an NMR experiment, can be used as input to obtain said spectrum on a computer via simulation.

In a preferred embodiment, the input interface is configured to receive a description input, wherein said system of differential equations describes a nuclear magnetic resonance spectrum of a substance. Additionally or alternatively, the system of differential equations is a system of linear, first-order differential equations. Such a system describes the behavior of molecules in an NMR experiment.

In a preferred embodiment, the input interface is configured to receive a description input including an accuracy parameter corresponding to a desired spectral resolution, in particular a spectral resolution corresponding to an obtainable spectral resolution of a nuclear magnetic resonance spectrum analysis system. This accuracy parameter may particularly be indicative of a total time of the NMR experiment, which is related to the obtainable resolution. In an experiment, the spectral resolution can also be influenced by other parameters in addition to the total time. Nevertheless, a finite spectral resolution usually means that only a finite time needs to be simulated.

In a preferred embodiment, the graph unit is configured to determine the graph based on iteratively including further vertices. Thereby, this iterative inclusion of further vertices may particularly correspond to add further vertices to the graph only if the respective expectation value or observable is considered relevant in view of the respective length parameter.

In a preferred embodiment, the distance unit is configured to determine the length parameter according to |*qₘₙ*|*^{α}* with |*qₘₙ*| corresponding to an absolute value of the respective coupling strength and α corresponding to a negative value, in particular according to . The length parameter is determined based on an inverse of an absolute value of a coupling strength as derived from the corresponding differential equation. The length parameter indicates a strength of the coupling. A stronger coupling in the differential equation results in a shorter distance in the graph (length parameter indicates a shorter length or a shorter distance).

In a preferred embodiment, the restriction unit is configured to determine for each vertex a shortest path between the start vertex and the respective vertex. Based on this, a length of the shortest path is calculated by adding up the length parameters along the shortest path; and remove vertices if the length of the shortest path exceeds a cutoff-radius determined based on the accuracy parameter. In other words, the length parameter can be interpreted as a length, i.e., a parameter that is directly indicative of a distance. Then, lengths (or distances) can be summed up in order to determine a path length in the graph. This forms the basis for determining whether a vertex is relevant or not.

In a preferred embodiment, the restriction unit is configured to calculate the length of the shortest path based on Dijkstra's algorithm. The use of Dijkstra's algorithm corresponds to a computational efficient approach for identifying a shortest path in a graph. An efficient calculation becomes possible.

In a preferred embodiment, the truncation unit is configured to remove the coupled expectation values corresponding to the removed vertices from the system of differential equations to obtain the truncated system of differential equations. The back transformation can particularly be based on leaving out terms of the original system that correspond to vertices that are determined to be located too far away from the start vertex.

In a preferred embodiment, the apparatus comprises an estimation unit for determining a computational complexity of solving the truncated system of differential equations. The estimation unit can thereby provide a parameter on a predefined scale. Based on this determined computational complexity, it can, e.g., be decided whether or not the simulation can be carried out on the available hardware. Also, it can be decided whether the use of a quantum computer could be necessary or beneficial.

In a preferred embodiment, the input interface is configured to receive a parameter representing available computational resources. The estimation unit is configured to determine whether the truncated system of differential equations can be solved with the available computational resources. The further input of such a parameter makes a direct comparison possible. It becomes possible to directly determine whether or not the simulation can be carried out with available hardware.

As used herein, a physical system may particularly refer to any system that can be described by a mathematical model based on physical laws, typically expressed in the form of differential equations. Such a system may particularly consist of particles, fields or other entities whose behavior evolves over time according to specified governing equations. One example is a quantum mechanical system governed by the Schrödinger equation. In particular, a physical system may correspond to the nuclear spin degrees of freedom in a molecule (or particle), whose spectrum is to be studied in an NMR experiment, i.e., the prediction of an NMR spectrum of a molecule. Physical System: A system that can be described by a mathematical model based on physical laws, typically expressed in the form of differential equations. Examples include quantum mechanical systems, electromagnetic systems, mechanical systems, and thermodynamic systems.

As used herein, a system of differential equations particularly corresponds to a set of equations involving derivatives of multiple dependent variables with respect to one or more independent variables used to model dynamic physical phenomena. A system of differential equations does not necessarily fully describe the system or the evolution of the observable of interest. In other words, the term does not exclude that the system of differential equations only includes a subset of a full set of differential equations corresponding to a complete description of the underlying physical system.

An expectation value of an observable particularly corresponds to a statistical average of a measurable physical quantity in a given state of a system. An expectation value can particularly be computed as the quantum mechanical expectation value using an operator formalism.

An accuracy of a simulation represents a degree to which a (numerical) simulation approximates the true behavior of the physical system, typically measured in terms of numerical error, stability, and convergence. An accuracy parameter can be indicative of one or more of these quantities.

A coupled expectation value or a coupled observable may particularly refer to an expectation value that depends on the evolution of multiple interrelated observables (expectation values of observables) due to interactions or correlations within the system (over time). As used herein, the term coupled particularly refers to expectation values being linked (coupled) via a differential equation.

Interacting observables may correspond to measurable quantities whose time evolution is influenced by their mutual interactions, often represented mathematically by commutator relations in quantum mechanics. In this context, the Liouville equation (also referred to as the von Neumann equation or the Liouville-von Neumann equation), which describes the time evolution of the density operator in quantum mechanics, expresses how the statistical state of a system evolves under the influence of the Hamiltonian, using the commutator with the Hamiltonian to encode the dynamical changes in observables.

Vertices in a graph can particularly correspond to elements or variables in a system. Vertices can sometimes also be referred to as sites or nodes. Connections (may also be referred to as edges) connect vertices and signify a relationship or interaction between the connected vertices, commonly used in graph-based representations of coupled differential equations. A connection can be attributed a length parameter which can particularly correspond to a length of the connection or a distance between the vertices.

A truncated system of differential equations can particularly correspond to a system of differential equations that has been approximated by neglecting some terms (particularly higher-order terms) or interactions beyond a certain threshold to reduce computational complexity. In the context of the present invention, such a truncated system of differential equations can particularly be obtained based on the graph and the vertices that were removed from the graph.

A Hamiltonian can particularly correspond to an operator representing the total energy of a physical system in quantum mechanics, governing the time evolution through the Schrödinger equation. A Hamiltonian can be used to derive a system of equations and thereby form part of a construction specification of a system of equations. Such a construction specification allows deriving a system of equations or parameters of a system of equations.

Nuclear magnetic resonance spectrum analysis is particularly a technique for determining the molecular structure of substances by analyzing the response of atomic nuclei to an applied magnetic field and radiofrequency pulses. In this context, the spectral resolution can particularly refer to the ability to distinguish between different frequency components in a spectrum, which can be crucial for resolving fine details in simulations and experimental measurements.

These and other aspects of the invention will be apparent from and elucidated with reference to the embodiment(s) described hereinafter. In the following drawings
- Figure 1: shows a schematic illustration of a simulation system according to the present invention;
- Figure 2: shows a schematic illustration of an apparatus according to the present invention;
- Figure 3: shows a schematic illustration of a workflow and possible usage of the concept of the present invention;
- Figure 4: shows an example for a graph determined by the apparatus of the present invention; and
- Figure 5: shows a schematic illustration of a method of the present invention.

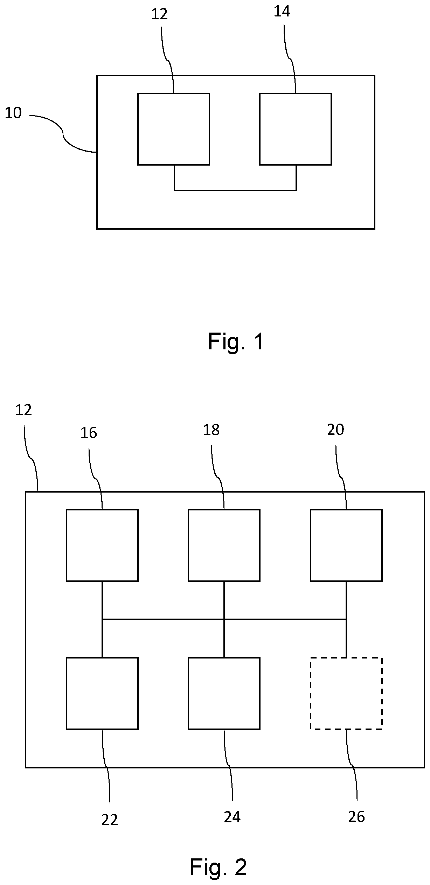

In figure 1, a simulation system 10 for simulating a physical system is schematically illustrated. The system 10 includes an apparatus 12 for preparing a simulation and a simulation unit 14. In the illustrated example, the apparatus 12 is particularly used for truncating a system of differential equations, which can then be solved by the simulation unit 14. The simulation system 10 can particularly correspond to a computer or a corresponding computer software. A relevant application is the prediction of a nuclear magnetic resonance spectrum of a substance.

In figure 2, an apparatus 12 for preparing a simulation of a physical system according to the present invention is schematically illustrated. The apparatus 12 includes an input interface 16, a graph unit 18, a distance unit 20, a restriction unit 22, a truncation unit 24 and an (optional) estimation unit 26. The different interfaces and units can be implemented in the form of hard- and/or software and can also be combined with one another upon need. The apparatus 12 is particularly suitable for determining a truncated system of differential equations which can form the basis for a sufficiently accurate simulation of a physical phenomenon.

The input interface 16 is configured to receive a description input. This input can particularly be the system of differential equations itself or a construction specification that allows deriving the system of differential equations therefrom. In particular, this construction specification can include a Hamiltonian along with an observable of interest. Further, a function can be received, in particular a Liouville-equation for quantum mechanical systems. Such a function may indicate how to determine a derivative with respect to time of an expectation value and, based thereupon, to which further expectation values this expectation value is coupled in a differential equation.

The graph unit 18 can particularly be configured to determine a graph based on the description input. For this, it is possible that vertices and connections are added iteratively from the start vertex according to the description input. The connections in the graph correspond to couplings in the differential equations.

The distance unit 20 is configured to attribute a parameter to a connection. In particular, a one-dimensional parameter can be determined that corresponds to a distance (on a predetermined scale). In other words, each connection is attributed with a distance or length of said connection. Thereby, this length is determined by making use of an inverse of an absolute value of a coupling strength in the differential equation.

The restriction unit 22 is then configured to remove (or disregard) vertices. In particular, vertices can be removed if the shortest path from the start vertex exceeds a predefined limit (also referred to as cutoff radius). This predefined limit can particularly correspond to the previously received (predefined) accuracy parameter. In other words, the predefined limit can be set depending on the required characteristics of the simulation.

The truncation unit 24 is configured to derive a truncated system of differential equations from the restricted graph. In particular, terms are removed from the system of differential equations for which the corresponding vertices in the graph have been removed.

The (optional) estimation unit 26 allows estimating a computational complexity of solving the truncated system of equations.

In a preferred embodiment, the approach of the present invention allows estimating the minimal resources needed to accurately perform a time evolution of a quantity (also referred to observable of interest) up to a given finite time. Thereby the time evolution is preferably governed by a linear, first-order system of differential equations of a number of degrees of freedom.

The approach of the present invention includes treating a system of equations based on a graph representation. One key feature is that the accuracy of the time evolution can be smoothly increase up to a certain time, by increasing the number of degrees of freedom of the calculation one at a time, starting with the most relevant degrees of freedom. The apparatus of the present invention can rank degrees of freedom of the system to be studied in order of relevance to the result. This procedure enables the study of various related quantities across systems of different size, investigate the convergence behavior of the time evolution up to a given time and make statements about the necessary number of degrees of freedom that need to be accounted for in order to perform an accurate calculation. Hence, e.g., the minimal necessary computational resources needed to perform a calculation can be judged by estimating the number of relevant degrees of freedom, but without the need to actually solve the system of equations (which might be computationally prohibitive). In particular, this feature is unique to the approach of the present invention compared to other methods which attempt to encapsulate the most important degrees of freedom of a calculation, e.g., tensor network methods.

There may be many reasons to focus on a finite evolution time. One may, e.g., intend to compare to: other computational methods that only solve up to certain time, data that is only captured to finite times, data where the time series decays over time (e.g., due to noise). Specifically, this can be important if one is interested in the spectral content of the time evolution. In this case, data with finite spectral resolution can be accurately represented by the Fourier transform of finite time data. In particular, this could be, e.g., data of NMR spectra of chemicals with finite spectral resolution, e.g., because it is measured data with limited experimental measurement accuracy, or computed spectra stemming from approximate methods with only a given accuracy (these could be from other calculations on conventional computers, or even quantum computations that have accuracy constraints, e.g., due to modeling errors, approximate algorithms, decoherence or a finite amount of measurement shots).

One direct consequence of estimating the minimal resource effort needed to perform a calculation using the approach of the present invention is the ability to judge whether a specific calculation is possible given certain hardware. This could be, e.g., to gauge if one can computationally reproduce an experimental NMR spectrum for analysis purposes, or if one can calculate a more accurate NMR spectrum than a reference calculation (on conventional or quantum computers). Hence, another consequence of this approach is by comparing to particular scenarios for a quantum computation (calculation on a quantum computer), if a quantum advantage is possible versus utilizing a given conventional computer.

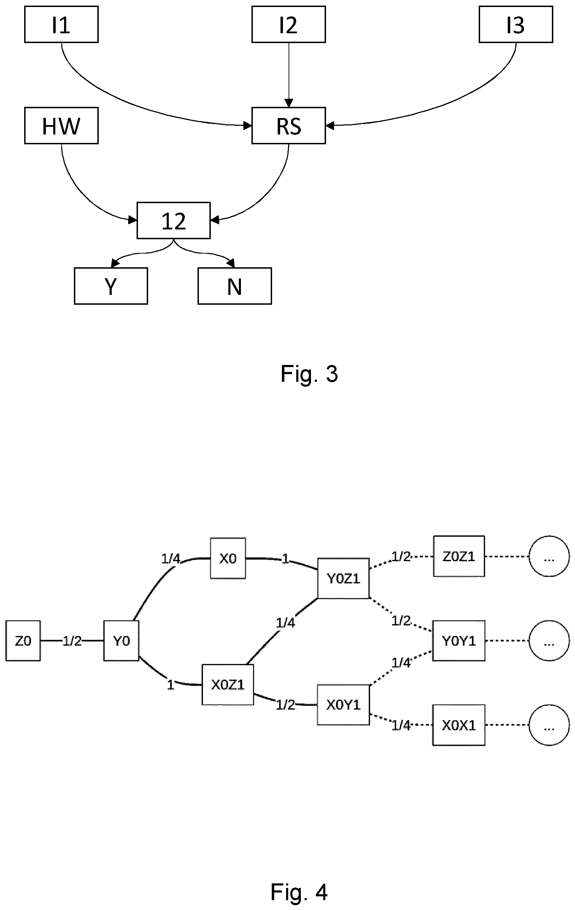

In this context, figure 3 schematically illustrates a flowchart that outlines an exemplary usage and workflow of the approach of the present invention in the context of NMR spectrum analysis. Thereby, I1 indicates a finite or decaying time data, I2 indicates a measured spectrum with finite resolution and I3 indicates an approximate calculation (conventional or quantum computer based). Based on these data, a finite spectral resolution RS can be derived corresponding to a desired (or required) accuracy of the simulation (accuracy parameter. Using this finite spectral resolution RS, the apparatus 12 of the present invention, in an embodiment, can allow determining that Y, on the one hand, the available hardware resources are sufficient to perform the calculation or, respectively, the reference spectrum to measured spectrum can be calculated, or, respectively a quantum calculation is inferior. Alternatively, it can be determined that N, on the other hand, more potent hardware is needed, or, respectively, the measured spectrum cannot be calculated to a desired accuracy, or, respectively a quantum calculation is superior.

In the following, details on the functionality of the approach of the present invention in preferred embodiments are outlined. The approach of the present invention particularly facilitates solving systems of linear, first-order differential equations, $\frac{d}{d t} {v}_{n} = {\sum }_{m} {A}_{nm} {v}_{m}$

Therein, A is an anti-Hermitian matrix with only off-diagonal elements. An example of such an equation might be a discrete version of the advection equation, $\frac{\partial }{\partial t} v \left(x, t\right) + c \left(x\right) \frac{\partial }{\partial x} v \left(x, t\right) = 0 ,$ which is often used to model the transport of a substance through the bulk motion of a fluid. However, a particular relevant application of the approach of the present invention is the prediction of NMR spectra.

More specifically, a quantum-mechanical Hamiltonian designed to model the nuclear spin degrees of freedom in a molecule, whose spectrum is to be studied in an NMR experiment, is considered. Such a model Hamiltonian takes the form $H = {\sum }_{i} {h}_{i} {σ}_{i}^{z} + {\sum }_{kl} {J}_{kl} {\vec{σ}}_{k} ⋅ {\vec{σ}}_{l} .$

The first term describes the interaction of each nuclear spin with the magnetic field of the NMR device, while the second term describes a Heisenberg spin-spin coupling between different nuclei. The fundamental object of interest which is to be studied in an NMR experiment is the spectral function ${{C}^{˜}}_{+ +} \left(ω\right) = {\int }_{- ∞}^{+ ∞} {C}_{+ +} \left(t\right) {e}^{- iωt} {e}^{- η \left|t\right|} dt$ which is the Fourier transform of the high-temperature correlation function, defined according to ${C}_{+ +} \left(t\right) = \frac{1}{D} Tr \left[{\left({M}^{+}\right)}^{†}{e}^{Lt}{M}^{+}\right] = \frac{1}{D} Tr \left[{M}^{-}{e}^{Lt}{M}^{+}\right]$ with ${M}^{\pm } = \frac{1}{2} \left({M}_{x}\pm {iM}_{y}\right) = \frac{1}{2} {\sum }_{i} {γ}_{i} \left({σ}_{i}^{x}\pm {iσ}_{i}^{y}\right) = {\sum }_{i} {γ}_{i} {σ}_{i}^{\pm } .$

**In the above** equation, 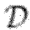 = 2*^{N}* is the Hilbert space dimension, 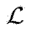 *=i*[*H,* ·] is the Liouvillian super-operator which evolves the density matrix of the system and *γᵢ* is the gyromagnetic factor for the nucleus at site i. The high-temperature correlation function can be interpreted as the "expectation value" of the operator *M⁻* as a function of time, when the system is placed in the initial "density matrix" *M*⁺.

The quantity *η* in the definition of the spectral function is a broadening scale, which represents a limited resolution in frequency space. This is because multiplying a function of time by a decaying exponential alters its Fourier transform by convolving it with a Lorentzian distribution, thus producing a "coarse-grained" version of the spectral function. In the context of an NMR experiment, such a limited frequency resolution is usually an artifact of the detector used to measure the spectral function in the experiment, and is difficult to remove in practice. In other contexts, such a broadening term may represent an actual decay in time due to noise in the system to be studied. Regardless of the origin of this broadening term, its presence in the definition of the spectral function implies that it is only necessary to perform the time evolution for a finite amount of time, depending upon the level of accuracy desired, since after a certain amount of time, the correlation function will ultimately just be multiplied by a quantity which is effectively zero, and will thus not contribute meaningfully to the integral that defines the Fourier transform (as an example, performing a time evolution out to a time 5.0 *η* might provide a reasonable level of accuracy, but still less accuracy than performing a time evolution out to a time 7.5 *η*).

It is noted that it is possible to mathematically rearrange the correlation function, so that it is given by ${C}_{+ +} \left(t\right) = {C}_{R} \left(t\right) + {iC}_{I} \left(t\right)$ with the real and imaginary correlation functions defined according to ${C}_{R} \left(t\right) = \frac{1}{2 D} {\sum }_{i} {γ}_{i} Tr \left[{σ}_{i}^{y}{e}^{Lt}{M}_{y}\right] ; {C}_{I} \left(t\right) = \frac{1}{2 D} {\sum }_{i} {γ}_{i} Tr \left[{σ}_{i}^{x}{e}^{Lt}{M}_{y}\right] .$

Mathematically, the problem is thus equivalent to studying the time evolution of the expectation values of all of the single-site *σ^{x}* and *σ^{y}* operators, starting from the initial state *M_{y}.*

In order to compute the time evolution of these expectation values as a practical matter (and therefore make a prediction for the result of the experiment), it is necessary to derive a set of differential equations describing the evolution of the system away from its initial state *ρ₀ = M_{y}.* If the state of the system at any other time is denoted ρ, then the Schrödinger equation means that the state evolves over time according to $\frac{d}{dt} ρ = Lρ = - i \left[H, ρ\right] .$

A convenient way to represent the state of the system is to write $ρ = \frac{1}{D} \left(I+{\sum }_{m} \left〈{A}_{m}\right〉 {A}_{m}\right) ; \left〈{A}_{m}\right〉 ≡ Tr \left[{A}_{m}ρ\right] .$

The operators {*Aₘ*} represent a complete set of all operators on the Hilbert space of the quantum-mechanical problem (which means that any other operator can be written as a unique linear combination of this set). If this is combined with the Schrödinger equation, the system of equations can be derived $\frac{d}{d t} \left〈{A}_{n}\right〉 = {\sum }_{m} {L}_{nm} \left〈{A}_{m}\right〉 ; {L}_{nm} = - \frac{i}{D} Tr \left[{A}_{n}^{†}\left[H, {A}_{m}\right]\right]$

The matrix L is always anti-Hermitian (in the case that the set {*Aₘ*} is chosen to be Hermitian, L will in fact always be real and anti-symmetric). Because of this, it is possible to rewrite the above equation in terms of a Hermitian matrix M, $\frac{d}{d t} {ϕ}_{n} = - i {\sum }_{m} {M}_{nm} {ϕ}_{m} ; {ϕ}_{n} ≡ \left〈{A}_{n}\right〉 , M ≡ + iL$

Since the Matrix M is Hermitian, the above equation can be interpreted as the evolution of the "wave function" *Φ* of a single quantum-mechanical particle which exists on a lattice of points, with each point corresponding to one of the operators in the set {*Aₘ*}*.* If this basis is chosen to only contain Hermitian operators, then the matrix *M* will not contain any diagonal entries, which corresponds to a single-particle problem with no on-site energy terms. In this single-particle interpretation of the problem, finding the *σ^{x}* and *σ^{y}* expectation values is equivalent to finding the value of the single-particle wave function on certain sites of the lattice. The initial state *M_{y}* corresponds in turn to a wave function which is localized only on the lattice sites corresponding to the σ*^{y}* operators.

The key methodology of the approach of the present invention includes the following: After mapping the problem to that of a single particle, the behavior of such systems can be used to determine effective approximation strategies. While quantum-mechanical systems evolve in a way which is, in principle, non-local, there is usually an effective propagation speed which limits the spread of the wave function, such that the wave function is exponentially suppressed outside of a certain horizon. This implies that the initially localized wave function can only reach certain sites within the finite evolution time, and so understanding the structure of the entire lattice is unnecessary.

To take advantage of this fact, the approach of the present invention focusses on solving for one expectation value (observable of interest) at a time (solving for all of the expectation values could be done in parallel, in order to save on computation time). As an example, it might be focused on finding the expectation value of ${σ}_{0}^{y}$*.* The goal of the approach of the present invention is to retain all sites in the lattice which are sufficiently "close" to this starting node, under the assumption that the information from sites "far away" will not have sufficient time to affect the wave function at the site of interest.

To determine the distance between two operator lattice sites which are directly connected by the system of equations, the information contained in the matrix M is used. The matrix element *Mₙₘ* corresponds (in an approximate sense) to the probability that the particle will make a single "jump" from site *n* to site m. One can therefore interpret this matrix element as leading to a direct path between sites n and m, with a "length" that should somehow vary inversely with the magnitude of *Mₙₘ* (so that a jump is more likely between "closer" sites). More specifically, to make sure that the distances are always positive, and also reflect the fact that the difficulty of the problem should depend on the relationship between the energy scales of the Hamiltonian and the broadening scale, it can be worked with the scaled couplings ${q}_{nm} = \left|{M}_{nm}\right| / η .$

This object has the additional advantage of being dimensionless. Then, a path length can be associated to each scaled coupling (length parameter), which should represent some sort of inverse relationship. One possible association for the path length is given by ${l}_{nm} = 1 / \sqrt{{q}_{nm}} ,$ although other associations are possible. The best possible association may depend on the particular problem at hand. Over time, the single particle can travel throughout the lattice, reaching sites that are not directly connected to the starting node. A path that stretches throughout the lattice (graph) is assigned a length which is equal to the sum of all of the individual connections that are traversed during the path. For any two sites in the lattice, we can assign a distance between them, according to the length of the shortest possible path that exists between them.

The goal of the approach of the present invention is to approximate the system of differential equations by retaining only the sites (vertices) within a certain distance, or radius, of the starting node (start vertex). This distance then represents the degree of approximation of the method - choosing larger radii typically results in a better approximation. To accomplish this, Dijkstra's algorithm can exemplarily be employed. Beginning with the starting node, Dijkstra's algorithm uses an iterative procedure to find all other sites which are less than the given radius away from the starting node, and is the most computationally efficient algorithm for accomplishing this task. In the process, the equations of motion between the degrees of freedom are also determined. Once the method has terminated, the equations of motion for the system are obtained, truncated to include only those degrees of freedom which are sufficiently "important", which is judged by their proximity to the starting node.

Similar methods exist for studying the approximate time evolution of quantum-mechanical systems. However, there are key differences between those methods and the approach of the present invention.

The approach of the present invention aims to study the system of differential equations relating the time evolution of different operator expectation values and then simplify it by removing certain "unimportant" operators (vertices in the graph). Several previous methods such as Rakovszky et al., "Dissipation-assisted operator evolution method for capturing hydrodynamic transport", 2020, White, "Effective dissipation rate in a Liouvillian-graph picture of high-temperature quantum hydrodynamics", 2023, Parker et al. "A universal operator growth hypothesis", 2019, and Hogben et al., "Spinach - a software library for simulation of spin dynamics in large spin systems", 2023, also aim to perform such an approximation. However, these methods determine the importance of operators using different criteria. This is because these methods are often focused on determining the long-time behavior of systems which undergo evolution towards a thermal equilibrium state. For this reason, these methods typically determine operators to be less important when the number of spins contributing to an operator is large, or when the operator has a large physical extent in the original real space lattice. Other methods attempt to reformulate the quantum-mechanical problem using a novel geometric interpretation based on ideas from quantum information theory, but these methods are also typically interested in studying long-time thermalization behavior.

Yet other approaches to this problem include the use of Tensor Network methods. However, these methods use an entirely different philosophy when making approximations to the time evolution and often struggle to provide good results for physical systems which exist in more than one (or sometimes two) dimensions.

The approach of the present invention presents several advantages over similar methods. By building up the lattice (graph) from the starting node, the solver focuses directly on the most important degrees of freedom controlling the time evolution. Because the method makes approximations in the space of operators, rather than in physical space, the degree of approximation can be tuned very finely, while avoiding unnecessary degrees of freedom. Results indicate that the accuracy of the approximate solution does indeed increase relatively smoothly with increasing radius, lending credence to the validity of this approach.

One issue facing approximation methods which are based in physical space rather than operator space is that these methods often include many unnecessary degrees of freedom because they account for all possible operators involving the retained sites, regardless of whether these operators actually contribute to the dynamics in a meaningful way. Another issue facing these methods is the question of on-site energy terms. When studying a typical NMR Hamiltonian, $H = {\sum }_{i} {h}_{i} {σ}_{i}^{z} + {\sum }_{kl} {J}_{kl} {\vec{σ}}_{k} ⋅ {\vec{σ}}_{l} ,$ an approximation method based in real space might interpret the couplings *Jₖₗ* as giving a notion of distance between spins. However, the dynamical evolution of the system will depend crucially on the relationship between the field terms and coupling terms, and it is not always clear how these terms can be incorporated into a notion of distance in real space. Because all of the terms in the Hamiltonian are treated on an equal footing in the space of operators, this complication does not exist for the approach of the present invention.

The approximation which is made by the approach of the present invention is intuitive to understand and simple to implement, and works for quantum systems in any number of spatial dimensions. It could also be applied to a wide range of other problems, whenever we have a system of differential equations that is described by an off-diagonal, anti-Hermitian matrix, and it is only desired to evolve for a finite amount of time.

The performance of the approach of the present invention is easy to quantify. In order to apply the method to, for example, an NMR problem, the three main computational bottlenecks involved in the method are a.) the computation of the matrix elements *Lₘₙ* by commuting basis operators with the Hamiltonian, b.) Dijkstra's algorithm and c.) the time evolution of the approximate equations of motion. The computational complexity of all three of these is well understood. In order to understand the feasibility of solving a given NMR problem classically, it is often not even necessary to perform the third time evolution step. For this reason, it is possible to develop a rigorous understanding of how increasing radius improves simulation accuracy, potentially allowing for an understanding of the error in the time evolution of a system for which comparison against an exact solution is not possible.

In the following, an example is given of how a graph in the approach of the present invention is determined based on a specific input and how, based on that graph, it is determined how the corresponding system of equations is to be truncated. In this respect it is referred to figure 4.

A Hamiltonian H, an initial state *ρ₀* (construction specification), a desired spectral resolution *η* (accuracy parameter) and an operator A (observable of interest), of which the time evolution should be calculated for, are provided. As an example, a transverse-field Ising model is chosen with just two sites, with the Hamiltonian $H = - h \left({Z}_{0}+{Z}_{1}\right) - g \left({X}_{0}+{X}_{1}\right) - {JZ}_{0} {Z}_{1} ,$ where it is denoted as *Xᵢ, Yᵢ,* Z the Pauli matrices on site *i.* To work with dimensionless units, for the example *η=1* is set.

Consider as the operator that it is desired to time evolve *A=Z₀.* The expectation value of an operator evolves according to the von Neumann equation via: $\frac{d}{d t} \left〈A\right〉 = i \left〈\left[H, A\right]\right〉 ,$ hence, in the present example this yields: $\frac{d}{d t} \left〈{Z}_{0}\right〉 = 2 g \left〈{Y}_{0}\right〉 .$

It is needed to continue taking the derivative of new operator averages that appear in the equations of motion, i.e., $\frac{d}{d t} \left〈{Y}_{0}\right〉 = - 2 g \left〈{Z}_{0}\right〉 + 2 h \left〈{X}_{0}\right〉 + 2 J \left〈{X}_{0}{Z}_{1}\right〉 ,$ next $\frac{d}{d t} \left〈{X}_{0}\right〉 = - 2 h \left〈{Y}_{0}\right〉 - 2 J \left〈{Y}_{0}{Z}_{1}\right〉 ,$ and $\frac{d}{d t} \left〈{X}_{0}{Z}_{1}\right〉 = - 2 J \left〈{Y}_{0}\right〉 - 2 h \left〈{Y}_{0}{Z}_{1}\right〉 + 2 g \left〈{X}_{0}{Y}_{1}\right〉 ,$ followed by $\frac{d}{d t} \left〈{Y}_{0}{Z}_{1}\right〉 = 2 J \left〈{X}_{0}\right〉 + 2 h \left〈{X}_{0}{Z}_{1}\right〉 - 2 g \left〈{Z}_{0}{Z}_{1}\right〉 + 2 g \left〈{Y}_{0}{Y}_{1}\right〉 ,$ and $\frac{d}{d t} \left〈{X}_{0}{Y}_{1}\right〉 = - 2 g \left〈{X}_{0}{Z}_{1}\right〉 - 2 h \left〈{Y}_{0}{Y}_{1}\right〉 + 2 h \left〈{X}_{0}{X}_{1}\right〉 ,$ and so on.

It can be seen how, e.g., *Z₀* and *Y₀* are connected by couplings of strength ±2g, *Y₀* and *X₀Z₁ by* ±2J, and so on (coupling strength). This is associated with distances $1 / \sqrt{\frac{\left|\pm 2g\right|}{η}}$ and $1 / \sqrt{\frac{\left|\pm 2J\right|}{η}} ,$ respectively (length parameter); and accordingly for the other operators. Setting in the example *h=8, g=2, and J=1*/*2,* the graph of figure 4 is obtained that depicts the operators and their distances:
If a cutoff radius of 2 is set (such a value would be set depending on the desired accuracy corresponding to the accuracy parameter), it would do the following: It starts at node *Z₀* and performs the commutation with the Hamiltonian, finding *Y₀* at distance 1/2. This distance is smaller than 2, so it keeps commuting to find new operators, e.g., *X₀,* which the shortest path to (in a graph with only *Z₀, Y₀,* and *X₀*) is 1/2 + 1/4 = 3/4 and therefore still smaller than the cutoff radius 2. The graph is built further until the three nodes *Z₀Z₁, Y₀Y₁,* and *X₀X₁* are hit.

Now, all nodes except these last three are closer than a distance 2 away from the starting node *Z₀* (start vertex), but for these three no path shorter (or equal) to 2 can be found. This means that these three, and also all nodes that would be found by further commuting with the Hamiltonian, would sit outside the chosen cutoff radius. Hence, these three nodes can be truncated and the approach of the present invention has determined that only the system of equations needs to be solved that contains the operators *Z₀, Y₀, X₀, X₀Z₁, Y₀Z₁,* and *X₀Y₁,* with the (now closed) linear system of equations to solve being: $\frac{d}{d t} \left〈{Z}_{0}\right〉 = 2 g \left〈{Y}_{0}\right〉 ,$ $\frac{d}{d t} \left〈{Y}_{0}\right〉 = - 2 g \left〈{Z}_{0}\right〉 + 2 h \left〈{X}_{0}\right〉 + 2 J \left〈{Z}_{1}\right〉 ,$ $\frac{d}{d t} \left〈{X}_{0}\right〉 = 2 h \left〈{Y}_{0}\right〉 - 2 J \left〈{Y}_{0}{Z}_{1}\right〉 ,$ $\frac{d}{d t} \left〈{X}_{0}{Z}_{1}\right〉 = - 2 J \left〈{Y}_{0}\right〉 - 2 h \left〈{Y}_{0}{Z}_{1}\right〉 + 2 g \left〈{X}_{0}{Y}_{1}\right〉 ,$ $\frac{d}{d t} \left〈{Y}_{0}{Z}_{1}\right〉 = 2 J \left〈{X}_{0}\right〉 + 2 h \left〈{X}_{0}{Z}_{1}\right〉 ,$ $\frac{d}{d t} \left〈{X}_{0}{Y}_{1}\right〉 = - 2 g \left〈{X}_{0}{Z}_{1}\right〉 ,$ with the initial conditions for each operator A being $\left〈O\left(t=0\right)\right〉 = Tr \left({O}_{{ρ}_{0}}\right) .$

Therein, *ρ₀* corresponds to the initial state or the initial density matrix. This system of equations (truncated system of equations) could now be solved using well-known computational methods, if the number of degrees of freedom (operators), that determine the computational complexity, are low enough such that this is feasible on a given hardware.

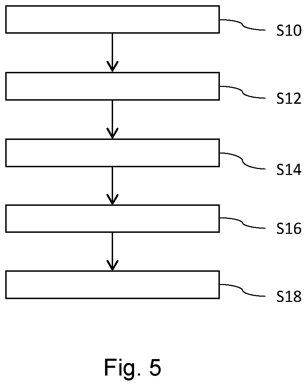

Figure 5 schematically illustrates a method of the present invention in a preferred embodiment. In particular, the method includes steps of receiving S10 a description input and an accuracy parameter, determining S12 a graph, determining S14 a length parameter, removing S16 vertices and deriving S18 a truncated system of differential equations. This method can particularly be implemented in the form of a software running on a conventional computer.

Thus, the foregoing discussion discloses and describes merely exemplary embodiments of the present disclosure. As will be understood by those skilled in the art, the present disclosure may be embodied in other specific forms without departing from the spirit or essential characteristics thereof. Accordingly, the description is intended to be illustrative, but not limiting the scope of the disclosure, as well as other claims. The disclosure, including any readily discernible variants of the teachings herein, defines, in part, the scope of the foregoing claim terminology such that no inventive subject matter is dedicated to the public.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

In so far as embodiments of the disclosure have been described as being implemented, at least in part, by software-controlled data processing apparatus, it will be appreciated that a non-transitory machine-readable medium carrying such software, such as an optical disk, a magnetic disk, semiconductor memory or the like, is also considered to represent an embodiment of the present disclosure. Further, such software may also be distributed in other forms, such as via the Internet or other wired or wireless telecommunication systems.

The elements of the disclosed devices, circuitry and system may be implemented by corresponding hardware and/or software elements, for instance appropriated circuits. A circuit is a structural assemblage of electronic components including conventional circuit elements, integrated circuits including application specific integrated circuits, standard integrated circuits, application specific standard products, and field programmable gate arrays. Further a circuit includes central processing units, graphics processing units, and microprocessors which are programmed or configured according to software code. A circuit does not include pure software, although a circuit includes the above-described hardware executing software.

## Claims

1. Apparatus (12) for preparing a simulation of a physical system, comprising:
an input interface (16) for receiving a description input including a system of differential equations or a construction specification of said system of differential equations, wherein said system of differential equations describes an evolution of an expectation value for an observable of interest of the physical system over time, and an accuracy parameter corresponding to a desired accuracy of the simulation;
a graph unit (18) for determining a graph based on the description input, wherein a start vertex of the graph represents an expectation value of the observable of interest, further vertices of the graph represent coupled expectation values and connections between the vertices represent couplings between the expectation values;
a distance unit (20) for determining a length parameter for each connection, said length parameter being determined based on an inverse of an absolute value of a coupling strength of the respective coupling;
a restriction unit (22) for removing vertices from the graph based on the length parameters of connections between the start vertex and the respective vertex and based on the accuracy parameter to obtain a restricted graph; and
a truncation unit (24) for deriving a truncated system of differential equations based on the restricted graph and the description input.

2. Apparatus (12) as claimed in claim 1, wherein the input interface (16) is configured to receive a description input including a Hamiltonian describing the physical system based on a plurality of interacting observables and an observable of interest of the plurality of interacting observables.

3. Apparatus (12) as claimed in claim 2, wherein the input interface (16) is configured to receive a description input including a quantum-mechanical Hamiltonian designed to model nuclear spin degrees of freedom in a molecule of which the spectrum is to be studied in a nuclear magnetic resonance spectrum analysis.

4. Apparatus (12) as claimed in any one of the preceding claims, wherein the input interface (16) is configured to receive a description input, wherein said system of differential equations
describes a nuclear magnetic resonance spectrum of a substance; and/or
is a system of linear, first-order differential equations.

5. Apparatus (12) as claimed in any one of the preceding claims, wherein the input interface (16) is configured to receive a description input including an accuracy parameter corresponding to a desired spectral resolution, in particular a spectral resolution corresponding to an obtainable spectral resolution of a nuclear magnetic resonance spectrum analysis system.

6. Apparatus (12) as claimed in any one of the preceding claims, wherein the graph unit (18) is configured to determine the graph based on iteratively including further vertices.

7. Apparatus (12) as claimed in any one of the preceding claims, wherein the distance unit (20) is configured to determine the length parameter according to |qₘₙ|^{α} with |qₘₙ| corresponding to an absolute value of the respective coupling strength and α corresponding to a negative value, in particular according to $\frac{1}{\sqrt{\left|{q}_{mn}\right|}}$.

8. Apparatus (12) as claimed in any one of the preceding claims, wherein the restriction unit (22) is configured to for each vertex
determine a shortest path between the start vertex and the respective vertex;
calculate a length of the shortest path by adding up the length parameters along the shortest path; and
remove vertices if the length of the shortest path exceeds a cutoff-radius determined based on the accuracy parameter.

9. Apparatus (12) as claimed in claim 8, wherein the restriction unit (22) is configured to calculate the length of the shortest path based on Dijkstra's algorithm.

10. Apparatus (12) as claimed in any one of the preceding claims, wherein the truncation unit (24) is configured to remove the coupled expectation values corresponding to the removed vertices from the system of differential equations to obtain the truncated system of differential equations.

11. Apparatus (12) as claimed in any one of the preceding claims, comprising an estimation unit (26) for determining a computational complexity of solving the truncated system of differential equations.

12. Apparatus (12) as claimed in claim 11, wherein
the input interface (16) is configured to receive a parameter representing available computational resources; and
the estimation unit (26) is configured to determine whether the truncated system of differential equations can be solved with the available computational resources.

13. Simulation system (10) for simulating a physical system, comprising an apparatus (12) as claimed in any one of the preceding claims and a simulation unit (14) for solving the truncated system of differential equations, wherein the system is preferably configured to predict a nuclear magnetic resonance spectrum of a substance.

14. Method for preparing a simulation of a physical system, comprising steps of:
receiving (S10) a description input including a system of differential equations or a construction specification of said system of differential equations, wherein said system of differential equations describes an evolution of an expectation value for an observable of interest of the physical system over time, and an accuracy parameter corresponding to a desired accuracy of the simulation;
determining (S12) a graph based on the description input, wherein a start vertex of the graph represents an expectation value of the observable of interest, further vertices of the graph represent coupled expectation values and connections between the vertices represent couplings between the expectation values;
determining (S14) a length parameter for each connection, said length parameter being determined based on an inverse of an absolute value of a coupling strength of the respective coupling;
removing (S16) vertices from the graph based on the length parameters of connections between the start vertex and the respective vertex and based on the accuracy parameter to obtain a restricted graph; and
deriving (S18) a truncated system of differential equations based on the restricted graph and the description input.

15. Computer program comprising program code means for causing a computer to perform the steps of the method according to the previous claim when said computer program is carried out on a computer.
